# EUROPEAN PATENT APPLICATION

(11) **EP 1 151 747 A1**
(43) Date of publication of application: **07.11.2001**
(21) Application number: 01110735.6
(22) Date of filing: 03.05.2001
(51) Int. Cl.: A61K 9/20, A61K 9/28, A61K 31/545

(54) **Modified release pharmaceutical formulations containing active principles having antibiotic activity**

(30) Priority: 04.05.2000 IT MI000972
(71) Applicant: LABORATORIO ITALIANO BIOCHIMICO FARMACEUTICO LISAPHARMA S.P.A., 22036 Erba (Como) (IT)
(72) Inventor: Maggi, Lauretta, 27100 Pavia (IT); Conte, Ubaldo, 21052 Busto Arsizio (IT)
(74) Representative: Gervasi, Gemma, Dr.

(57) **Abstract**

Formulations based on substances endowed with antibiotic activity for the preparation of oral hydrophilic matrixes (modified release tablets). Said hydrophilic matrixes can release the carried antibiotic gradually and following a release profile which can be established by suitable in vitro tests. An essential feature of said hydrophilic matrixes consists in that they can provide a high bioavailability characterized by such extended effective plasmatic levels to enable only one or two daily administrations, thus simplifying dosage and correct use by the patient.

## Description

### State of the art and prior art

In recent years modified release (extended release) pharmaceutical forms, and in general the so-called therapeutic systems releasing the active substance they carry in an extended period of time, possibly pre-programmable by means of suitable in vitro tests, have acquired more and more importance.

Such a result, allowing to reduce the number of daily administrations, can be reached by means of several methods requiring, in any case, the transmission of a high amount of active substance which, when the system works correctly, is gradually released by said system thus carrying out its activity for an extended period of time.

Particularly in the pharmaceutical field there is a stronger need to be provided with "systems" releasing their active substance, i.e. a drug, for an extended period of time and with a suitable speed establishing and keeping given plasmatic levels during the therapeutic period, therefore effective but not toxic. The longer the effective period provided for the system is (that is, the more extended the release of the active principle should be), the higher is the amount of active substance which must be carried in the system or pharmaceutical formulation.

Until now there have been several embodiments which have been carried out and marketed so as to obtain an extended "therapeutic coverage", i.e. to maintain therapeutically effective plasmatic levels of the active substance. In the biomedical field, above all for humans, a great development has characterized systems releasing the active substance at constant speed and for an extended period of time with the aim to find "in vivo" the same or similar trend. Indeed, said systems are designed to determine "in vivo" a kinetic pattern for a release known as zero order, which allows in many cases to maintain constant in time the hematic level of the drug transmitted through the pharmaceutical form.

Therapeutic systems which have had a broad spreading are the so-called "reservoir" systems, OROS osmotic pumps, as described in US Patent no. 4.160.020 (1979), and "push-pull" systems.

The above-mentioned embodiments are indubitably advantageous since they enable a strong simplification of dosage schemes with administrations which are often to be carried out only once a day, also obtaining a higher compliance and acceptance of the prescribed therapy by the patient.

These devices, however, if not working correctly, for instance because of production changes, involve several limitations and can cause serious inconveniences, both structural and functional, severely limiting their use and/or advising against it.

In said systems the adjustment of the release program for the transmitted active principle is obtained by means of an osmotic gradient, through a graduated hole made on the semi-permeable polymeric shell coating the system. Said shell is rigid and not biodegradable and its accidental abrasion or breaking involves the release in very short times of high amounts of active substance (drug), thus causing a rapid absorption and, consequently, high hematic levels, which phenomenon is known as "dose dumping".

Said therapeutic systems are presently used in practice for the treatment of chronic diseases; as a matter of fact, they transmit anti-hypertension drugs, calcium-antagonists, anti-arrhythmia drugs normally requiring low dosages and therefore allowing to obtain therapeutic systems having weight and dimensions acceptable for the patient.

A great limitation to such osmotic systems is connected with the dose of active principle which can be transmitted; as a matter of fact, when the drug dose increases, the dimensions of the system increase, even highly. For instance, in order to transmit through an osmotic system 90 mg of niphedipine it is necessary to use a great amount of technological adjuvants, such as osmotic agents, until a therapeutic system (OROS film-coated tablet) weighing 1.300 mg is obtained. This makes the pharmaceutical form little acceptable for the patient, very often an old person, since swallowing is difficult.

The aforesaid problem is particularly critical if it is necessary to administer orally high doses of the drug; this situation occurs especially in case of antibiotics, for most of which a dosage above 500 mg is required.

Among the most spread antibiotics, physicians are now addressing a particular attention to the class of cephalosporins.

Among all drugs belonging to this class, a particular attention is deserved by cefaclor, which is indicated and widely used in the treatment of infections strengthened by sensitive germs, such as infections of the respiratory system, including pneumonia, bronchitis, recurrent bronchitis and new acute phases of chronic bronchitis, tonsillitis, pharyngitis, sinusitis, otitis media. It is also employed in the treatment of infections of skin and soft tissues beyond infections of the genital-urinary system including cystitis, pielonephritis and gonococcus or nonspecific urethritis. The main action mechanism of cephalosporins reveals itself through the inhibition of the synthesis of the cell wall of the pathogenic microorganism. Cefaclor is usually administered orally as granulate bags to be dispersed in water and as ready-release tablets.

Recently modified release pharmaceutical forms for oral administration, commonly known as retard forms, have been proposed and marketed for this drug.

Among these a particular attention should be addressed to a medicinal speciality containing 750 mg of cefaclor, based on the formulation for oral use described in US patent no. 4.968.508, in which it is claimed that the critical factor to obtain a good absorption and a high bioavailability of cefaclor is the use of particular gastro-resistant entero-soluble polymeric materials based on acrylic polymers in a percentage of 5 to 29% referred to the tablet weight. In order to promote the absorption of cefaclor, the absolute necessity to use polymeric materials which are insoluble in gastric liquids, in a percentage up to 30%, is claimed by US Patent 4968508, too.

In the light of the formulations described in these patents a medicinal speciality (Panacef RM) has been developed and marketed in Italy and Europe, said drug being a reference for every other speciality based on cefaclor which should be registered.

### Description of the invention

We have now surprisingly found, which is the object of the present patent application, that using particular hydrophilic polymers or mixtures of said hydrophilic polymers, in combination with other technological adjuvants, it is possible to obtain pharmaceutical forms for oral administration containing cephalosporins defining a good bioavailability characterized by such extended effective plasmatic levels to enable only one or two daily administrations. Said pharmaceutical forms, which can be classified as hydrophilic matrixes, are film-coated lenticular tablets releasing the contained antibiotic gradually and following a release pattern which can be pre-established by means of suitable in vitro tests.

The essential feature of said hydrophilic matrixes consists in that after administration to healthy volunteers they show a high bioavailability confirmed by the statistic evaluation of pharmaco-kinetic parameters such as Cmax, tmax and AUC, as will be better described in the examples contained in the present application.

Even more surprisingly we have found that the formulations claimed in the present patent application allow to obtain plasmatic levels and a total bioavailability which are even higher that those of the formulations which are already registered and indicated as references.

This result enables a simplification of dosage, simplifies correct use by the patient and increases its acceptability.

The composition described contains, beyond the antibiotic, hydrophilic polymers involving a modulation in the release of the transmitted active principle according to a release kinetic pattern which can be pre-programmed "in vitro" using suitable control methods.

The formulation of the hydrophilic matrix described above can be usefully employed in all those cases requiring a gradual release of the active substance so as to ensure an extended effective therapeutic coverage. Said requirement is particularly necessary in case of antibiotic therapy: so that an antibiotic performs its activity it is essential to attain a Minimal Inhibiting Concentration (MIC) (plasmatic) obviously depending on the sensitivity of the pathogenic agent to be treated.

The formulation of the hydrophilic matrixes described in the present patent application is able to avoid the disadvantages and to overcome the limitation described above, connected with the preparation and administration of osmotic therapeutic systems (osmotic pumps).

We have unexpectedly found that by using consolidated pharmaceutical technologies having a high industrial productivity it is possible to produce hydrophilic matrixes which do not give rise to "dose dumping" and from which the antibiotic is gradually released.

As a matter of fact, we found, which is a peculiar feature of the present invention, that the beginning and the speed, and therefore the length of the release of the active substance, on the basis of in vitro tests, can be suitably programmed according to the specific therapeutic needs required for the antibiotic.

The formulation of the hydrophilic matrixes of the invention is characterized in that for its preparation, beyond the active principle, hydrophilic pharmaceutically acceptable substances are used, which can modulate (slacken) the antibiotic release.

Active substances transmitted through the hydrophilic matrix can include, beyond cefaclor, cephamandol and cephalosporinic derivatives generally classifiable as belonging to first, second or third generation, which can be used for the therapy of infections nourished by sensitive germs.

Among the most widely used antibiotics which can be transmitted through the pharmaceutical form, a particular attention is presently addressed by physicians to the class of cephalosporins, drugs classified as belonging to the first generation including cephalotin, cephacetryl, cephapyrin, cephaloridin, cephalozin, cephalo-glycine, cephalexin, cephadroxyl, cephachlor, cephadrin, cephatrizin, cephroxadin, cephuroxime axetil; to the second generation of cephalosporins belong, among others, cephamandol, cephonicid, cephuroxime, cephmetazol, moxalactam; to the third generation including cephotaxime, cephoperazone, cephoranid, cephpyramid, cephuzonam.

Natural and/or synthetic polymeric materials belonging to the class of the so-called gellable and/or erodible hydrophilic polymers which can control (retard) the active agent release in the tablets described in the present patent application comprise among others polyvinyl pyrrolidone (non cross-linked), carboxymethyl cellulose and its alkali metal or alkali-earth metal salts, carboxyvinyl polymers, hydroxypropylmethyl cellulose, polyvinyl alcohols, mannans, galactomannans, glucanes, scleroglucanes, carragenanes, pectine, xanthanes, agar, pullulane, poloxamers, polyoxyethylen glycols with different molecular weights, chitine, chitosanes and derivatives, beta cyclodextrin and derivatives of dextrins in general. Said polymeric substances constitute 5 to 90% of the tablet weight, preferably 10 to 50%, depending on the amount and solubility of the carried active substance.

Depending on the characteristics of the active agent carried, on its concentrations and solubility, the choice of a specific polymer for the preparation of the hydrophilic matrixes is determined by the particular chemical and physical properties and by the solubility and gelation properties.

In particular, it is possible to use various types of hydroxypropylmethyl cellulose having different molecular weights (1.000 to 4.000.000) and different substitution degrees, with differentiated features, mainly erodible or mainly gelable, according to viscosity and substitution degree (S.D.) in the polymer chain. Particularly interesting results can be achieved with hydroxypropylmethyl cellulose with a viscosity in standard aqueous solution comprised in the range 5 ÷ 15.000 cP., such as for instance the products known under the trade name Methocel E 50 LV Colorcon and Methocel K 4 M Colorcon having a suitable gelation degree.

The present patent application is characterized in that in the preparation of the hydrophilic matrix a single type of hydrophilic polymer can be used, or mixtures of hydrophilic polymers having different gelation and/or erosion features. In particular, a single erodible polymer or a mixture of erodible hydrophilic polymer and gelable hydrophilic polymer can be used, in which case the relation between erodible polymer and gelable polymer can vary between 15:1 and 1:10, preferably between 10:1 and 1:5. Such a mixture comprises hydroxypropyl-methyl cellulose having an average molecular weight of about 20.000 and a molecular weight of about 86.000, such as the products known under the trade name Methocel E 50 LV Colorcon and Methocel K 4 M Colorcon, having viscosity in standard aqueous solution, respectively of 50 and 4.000 cP.

As is well known to the person skilled in the art, in the preparation of the hydrophilic matrixes constituting the object of the present patent application, excipients can be used which are normally used in pharmaceutical technology, such as mannitol, lactose, sorbitol, xylitol, talc, magnesium stearate, colloidal silica and others like glyceryl monostearate, hydrogenated castor oil.

The practical result which can be achieved with the hydrophilic matrixes claimed in the present invention is the programmed release of the transmitted active principle. It is therefore possible to transmit a suitable amount of drug, thus avoiding the phenomenon of dose dumping and satisfying specific therapeutic needs with the controlled release of the transmitted active principle.

Through a suitable formulation of the core and of suitable methods for in vitro control it is possible to program the release of the active substance with fairly high precision.

Other adjuvant can also be employed, such as those chosen in the group comprising the classes of substances usually employed in pharmaceutical technology, such as diluents, buffer agents, binders, adsorbents, etc. and particularly, starch, pre-geled starch, calcium phosphate, mannitol, lactose, saccharose, glucose, sorbitol, microcrystalline cellulose and binding agents such as gelatin, polyvinyl pyrrolidone, methyl cellulose, starch water, ethyl cellulose, acacia and tragacanth.

Moreover, it is possible to use as adjuvants other excipients normally employed in pharmaceutical technology such as magnesium stearate, stearic acid, colloidal silica, glyceryl monostearate, polyoxyethylen glycols with a molecular weight of 400 to 50.000, hydrogenated castor oil, waxes and mono-, bi- and tri-substituted glycerids.

Moreover, it is possible to employ diluents, binders, lubricants, buffer agents, anti-adhesive agents, gliding agents and other substances known to the person skilled in the art, which can give the tablet the desired features, as will be better explained in the following examples.

The tablets according to the invention can be produced starting from mixtures of powders or granulates using manufacturing technologies currently used, and therefore with a manufacturing process which can be transferred directly onto an industrial scale.

Said tablets are preferably sphere-shaped or lenticular, so as to enable the following film-coating process.

The hydrophilic matrixes obtained undergo preferably a coating process allowing to obtain a uniform and continuous coating on the whole tablet surface, said coating being applied with the sole intent to hide the bitter taste of the active substance.

The film-coating can be carried out according to known methods (in a turning tray, in a fluidized bed and/or with other methods), with a layer (film or membrane) of soluble polymeric material or permeable to water and/or aqueous fluids.

As coating agents it is possible to employ solution and/or aqueous dispersion of synthetic and/or semi-synthetic natural polymers of cellulose, of hydroxypropyl-methyl cellulose and its derivatives; other products can be used, which however should not affect the release kinetic pattern of the active agent transmitted through the matrix.

A useful commercial product is the hydroxypropylmethyl cellulose Pharmacoat 606 of Shinetzu.

In the filming phase, beyond the coating polymeric material with the function to hide the bitter taste of the active substance transmitted through the core, plasticizing substances can also be used, which give said coating suitable adhesion and resistance features, and also colorants and opacifiers.

The plasticizers which can be used comprise polyoxyethylen glycols with molecular weight of 400 to 50,000, castor oil, silicon derivatives such as simeticone, diethyl phtalate, triethyl citrate, tributyl citrate, triacetine, dibutyl sebacate.

If the hydrophilic matrix is irregularly pigmented, for instance because of a colored active agent, it is possible to use dyes and/or pigments, pharmaceutically acceptable iron oxides and opacifiers such as talc, kaolin and titanium dioxide.

The polymeric coating constitutes 0.2% to 20%, preferably 3%-15%, of the total weight of the finished tablet.

### Example 1

Preparation of a series of (5,000) coated tablets containing Cefaclor monohydrate corresponding to 750 mg of anhydrous Cefaclor. The preparation comprises a first stage in which lenticular tablets (cores) are manufactured, onto which a film of water soluble polymeric coating is then applied.

### Example 1-a

Preparation of a series of 5,000 hydrophilic matrixes (uncoated tablets) containing Cefaclor monohydrate corresponding to 750 mg of anhydrous Cefaclor Each tablet shows the following individual composition:

| | |
|---|---|
| Cefaclor monohydrate | 786.720 mg |
| (Batch ACS DOBFAR 660200 048 9) corresponding to 750,0 mg of anhydrous Cefaclor | |
| Hydroxypropylmethyl cellulose **(Methocel E 50 LV, Colorcon, UK)** | 125.00 mg |
| Hydroxypropylmethyl cellulose **(Methocel K4M, Colorcon, UK)** | 25.00 mg |
| Mannitol (CFM Batch no. 9066G/400) | 72.780 mg |
| Polyvinyl pyrrolidone (Povidone K 30, ISP, Wayne, USA) | 60.000 mg |
| Magnesium stearate (USP grade, C. Erba, Milan -I) | 12.000 mg |
| Colloidal silica (Syloid 244 Grace, Worms, D) | 3.000 mg |
| Total | 1084.500 mg |

Cefaclor, mixed in a Turbula device for 10 minutes with Methocel E 50 LV, Methocel K4M and mannitol, is granulated with a 20% solution of PVP (polyvinyl pyrrolidone) in water. The wet mass is then passed on a 16-mesh sieve, dried in an oven at 40°C for two hours, then the granulate is calibrated on a 25-mesh sieve and dried in an oven until a constant weight is reached.

The granulate thus obtained is charged with magnesium stearate and colloidal silica and mixed in a Turbula mixer for 15 min.

Divisible tablets weighing 1084.50 mg have been prepared by using a capsule-type punch sized 22 x 11 mm (upper punch with notch).

### Example 1-b

Control of the release features on hydrophilic matrixes (uncoated tablets) containing Cefaclor monohydrate corresponding to 750 mg anhydrous Cefaclor. The (uncoated) tablets obtained undergo a dissolution test according to USP 23 operating under the following conditions:
- Apparatus 1 basket (100 rpm),
- Apparatus 2 paddle (100 rpm),
- means: 900 ml of 0,1 N hydrochloric acid, 37°C,
- spectrophotometric evaluation at 264 nm.

Table 1° shows the data referring to the release of the active agent from the uncoated hydrophilic matrixes, obtained with the basket method and with the paddle method.

**TABLE I°**

| Time (hrs) | % released basket | % released paddle |
|---|---|---|
| 0.5 | 14.2 | 14.7 |
| 1 | 26.3 | 33.7 |
| 2 | 55.6 | 61.0 |
| 3 | 78.8 | 81.2 |
| 4 | 95.4 | 96.2 |
| 5 | 100.8 | 101.3 |
| 6 | 101.2 | 101.8 |

It should be observed that the active principle is released from the coated tablets gradually in a time interval of about 5-6 hours.

### Example 1-c

Coating of the hydrophilic matrixes containing Cefaclor monohydrate corresponding to 750 mg of anhydrous Cefaclor.

The percentage composition of the suspension used for the coating is the following:

| | |
|---|---|
| Hydroxypropylmethyl cellulose (Pharmacoat 606 Shinetzu J.) | 7.556 % |
| Propylen Glycol (C. Erba) | 0.444 % |
| Titanium Dioxide (C. Erba) | 1.800 % |
| Distilled water | 92.200 % |
| Total | 100.000 % |

Hydroxypropylmethyl cellulose (Pharmacoat 606 Shinetzu J.) dissolves in water under agitation. The clear solution thus obtained is charged with propylene glycol and eventually titanium dioxide. The suspension obtained is homogenized under agitation with Ultra-Turrax for 10 min.

Then the film-coating stage is carried out in a turning tray using an automatic spraying system (1.00 mm nozzle-operating pressure 1.1 atm).

The coating process goes on until about 15.50 mg of coating is applied onto each tablet.

Therefore, on each tablet the following amount of coating is applied:

| | |
|---|---|
| Hydroxypropylmethyl cellulose (Pharmacoat 606 Shinetzu J.) | 12.022 mg |
| Propylen Glycol (C. Erba) | 0,688 mg |
| Titanium Dioxide (C. Erba) | 2.790 mg |
| Total | 15.500 mg |

Therefore, the total composition of the coated tablets is the following:
Each tablet shows the following individual composition:

| | |
|---|---|
| Cefaclor monohydrate (ACS DOBFAR) | 786.720 mg |
| corresponding to anhydrous Cefaclor750 mg Hydroxypropylmethyl cellulose **(Methocel E 50 LV, Colorcon, UK)** | 125.00 mg |
| Hydroxypropylmethyl cellulose **(Methocel K4M, Colorcon, UK)** | 25.00 mg |
| Mannitol (CFM Batch no. 9066G/400) | 72.780 mg |
| Polyvinyl pyrrolidone (Povidone K 30, ISP, Wayne, USA) | 60.000 mg |
| Magnesium stearate (USP grade, C. Erba, Milan -I) | 12.000 mg |
| Colloidal silica (Syloid 244 Grace, Worms, D) | 3.000 mg |
| Hydroxypropylmethyl cellulose **(Pharmacoat 606 Shinetzu J.)** | 12.022 mg |
| Propylen Glycol (C. Erba) | 0.688 mg |
| Titanium Dioxide (C. Erba) | 2.790 mg |
| Total | 1100.000 mg |

### Example 1-d

Control of the release features on coated hydrophilic matrixes (coated tablets) containing Cefaclor monohydrate corresponding to 750 of anhydrous Cefaclor.

The coated tablets obtained undergo a dissolution test according to USP 23 operating under the conditions described in example 1b:
Table II° shows the data referring to the release of the active agent from the coated hydrophilic matrixes, obtained with the paddle method and with the basket method.

**TABLE II°**

| Time (hrs) | % released basket | % released paddle |
|---|---|---|
| 0.5 | 14.4 | 14.5 |
| 1 | 26.2 | 33.0 |
| 2 | 54.9 | 60.7 |
| 3 | 78.2 | 80.8 |
| 4 | 95.9 | 96.4 |
| 5 | 99.8 | 100.6 |
| 6 | 101.4 | 101.3 |

It should be observed that the active principle is released from the coated tablets gradually in a time interval of about 5 hours.

Comparing the data contained in Table I° and Table II° it is evident that the application of the coating film does not modify the release speed in the coated tablet with respect to the uncoated tablet.

### Example 1-e

Results of the bioavailability tests carried out on 6 healthy volunteers after administration of the coated hydrophilic matrixes (coated tablets) containing Cefaclor monohydrate corresponding to 750 mg of anhydrous Cefaclor, as described in example 1.

In particular, the pharmaco-kinetic profile of the preparation described in example 1-d was evaluated by using as reference drug the medicinal speciality Panacef RM (Ely Lilly Italia) - batch no. 52664Y1, expiry date 04.2002, containing 750 mg of cefaclor.

The study was carried out in open, single dose, two periods, two sequences, two treatments, randomized, balanced and under crossover.

The testing, carried out on six people (healthy volunteers), consisted in the oral administration of the preparation described in the example 1-d and, after a washout time, of the reference drug having the same content of active principle.

The test results are shown in Table III°:

**TABLE III°**

| | example 1 d | Panacef RM |
|---|---|---|
| Cmax | 5.37 (s.d. 1.43) | 6.26 (s.d. 1.56) |
| tmax (hrs) | 2.25 (s.d. 0.82) | 1.67 (s.d. 0.75) |
| AUC₀₋ₜ | 20.11 (s.d. 4.61) | 19.80 (s.d. 2.98) |

On the basis of the international evaluation criteria accepted by health authorities, from the analysis of the data obtained it should be observed that the preparation described in example 1 of the present patent application is biologically equivalent to the reference drug.

### Example 2

Preparation of a series of (5,000) coated tablets containing Cefaclor monohydrate corresponding to 750 mg of anhydrous Cefaclor. The preparation comprises a first stage in which lenticular tablets (cores) are manufactured, onto which a film of water soluble polymeric coating is then applied.

### Example 2-a

Preparation of a series of 5,000 hydrophilic matrixes (uncoated tablets) containing Cefaclor monohydrate corresponding to 750 mg of anhydrous Cefaclor.

Each tablet shows the following individual composition:

| | |
|---|---|
| Cefaclor monohydrate (Batch ACS DOBFAR 660200 048 9) | 786.720 mg |
| corresponding to 750,0 mg of anhydrous Cefaclor Hydroxypropylmethyl cellulose | |
| **(Methocel E 50 LV, Colorcon, UK)** | 150.00 mg |
| Mannitol (CFM Batch no. 9066G/400) | 72.780 mg |
| Polyvinyl pyrrolidone (Povidone K 30, ISP, Wayne, USA) | 60.000 mg |
| Magnesium stearate (USP grade, C. Erba, Milan -I) | 12.000 mg |
| Colloidal silica (Syloid 244 Grace, Worms, D) | 3.000 mg |
| Total | 1084.500 mg |

Cefaclor, mixed in a Turbula device for 10 minutes with Methocel E 50 LV and mannitol, is granulated with a 20% solution of PVP in water. The wet mass is then passed on a 16-mesh sieve, dried in an oven at 40°C for two hours, then the granulate is calibrated on a 25-mesh sieve and dried in an oven overnight until a constant weight is reached.

The granulate thus obtained is charged with magnesium stearate and colloidal silica and mixed in a Turbula mixer for 15 min.

The granulate shows good flow properties and can be used to prepare divisible tablets weighing 1084.50 mg by using a capsule-type punch sized 22 x 11 mm and a Ronchi compressing machine.

### Example 2-b

Control of the release features on hydrophilic matrixes (uncoated tablets) containing Cefaclor monohydrate corresponding to 750 mg of anhydrous Cefaclor. The (uncoated) tablets obtained undergo a dissolution test according to USP 23 operating under the conditions described in example 1-b:
Table IV° shows the data referring to the release of the active principle from the uncoated hydrophilic matrixes, obtained with the basket method and with the paddle method.

**TABLE IV°**

| Time (hrs) | % released basket | % released paddle |
|---|---|---|
| 0.5 | 16.2 | 20.0 |
| 1 | 32.3 | 42.8 |
| 2 | 64.2 | 81.1 |
| 3 | 90.4 | 99.6 |
| 4 | 100.2 | 101.2 |
| 5 | 100.4 | 101.6 |

It should be observed that the active principle is released from the uncoated tablets in about 3-4 hours.

### Example 2-c

Coating of the hydrophilic matrixes containing Cefaclor monohydrate corresponding to 750 mg of anhydrous Cefaclor.

The film-coating stage is carried out according to the method and the procedures indicated in example 1-c. The coating process goes on until about 15.50 mg of coating is applied onto each tablet.

Therefore, the total composition of the coated tablets is the following:
Each tablet shows the following individual composition:

| | |
|---|---|
| Cefaclor monohydrate (ACS DOBFAR) | 786.720 mg |
| corresponding to 750 mg of anhydrous Cefaclor Hydroxypropylmethyl cellulose (Methocel E 50 LV, Colorcon, UK) | 150.000 mg |
| Mannitol (CFM Batch no. 9066G/400) | 72.780 mg |
| Polyvinyl pyrrolidone (Povidone K 30, ISP, Wayne, USA) | 60.000 mg |
| Magnesium stearate (USP grade, C. Erba, Milan -I) | 12.000 mg |
| Colloidal silica (Syloid 244 Grace, Worms, D) | 3.000 mg |
| Hydroxypropylmethyl cellulose | |
| (Pharmacoat 606 Shinetzu J.) | 12.022 mg |
| Propylen Glycol (C. Erba) | 0.688 mg |
| Titanium Dioxide (C. Erba) | 2.790 mg |
| Total | 1100.000 mg |

### Example 2-d

Control of the release features on coated hydrophilic matrixes (coated tablets) containing Cefaclor monohydrate corresponding to 750 mg of anhydrous Cefaclor.

The coated tablets obtained undergo a dissolution test according to USP 23 operating under the conditions described in examples 1b and 2b:
Table V° shows the data referring to the release of the active principle from the coated hydrophilic matrixes, obtained with the paddle method and with the basket method.

**TABLE V°**

| Time (hrs) | % released basket | % released paddle |
|---|---|---|
| 0.5 | 15.8 | 19.7 |
| 1 | 31.7 | 41.9 |
| 2 | 65.0 | 80.8 |
| 3 | 88.9 | 98.5 |
| 4 | 101.0 | 100.3 |
| 5 | 101.6 | 102.0 |

Comparing the data contained in Table IV° and Table V° it is evident that the application of the coating film does not modify the release speed in the coated tablet with respect to the uncoated tablet.

### Example 2-e

Results of the bioavailability tests carried out on 6 healthy volunteers after administration of the coated hydrophilic matrixes (coated tablets) containing Cefaclor monohydrate corresponding to 750 mg of anhydrous Cefaclor, as described in example 2.

In particular, the pharmaco-kinetic profile of the preparation described in example 2-d was evaluated by using as reference drug the medicinal speciality Panacef RM (Ely Lilly Italia) - batch no. 52664Y1, expiry date 04.2002, containing 750 mg of cefaclor.

The study was carried out in open, single dose, two periods, two sequences, two treatments, randomized, balanced and under crossover.

The testing, carried out on six people (healthy volunteers), consisted in the oral administration of the preparation described in the example 2-d and, after a washout time, of the reference drug having the same content of active principle.

The test results are shown in Table VI°:

**TABLE VI°**

| | example 2 d | Panacef RM |
|---|---|---|
| Cmax | 6,10 (s.d. 1.1) | 4,93 (s.d. 1.5) |
| tmax (hrs) | 2.10 (s.d. 1.1) | 2.60 (s.d. 1.5) |
| AUC₀₋ₜ | 18.62 (s.d. 3.2) | 15.06 (s.d. 5.0) |

On the basis of the international evaluation criteria accepted by health authorities, from the analysis of the data obtained it should be observed that the preparation described in example 2-d of the present patent application shows a higher bioavailability than the reference drug: AUC 0-t 18.62 versus 15.06. Moreover Cmax (6.10 ng/ml versus 4.93 ng/ml) and tmax (2.10 hrs versus 2.60) values show an improvement with respect to the reference drug (registered medicinal speciality already mentioned above). In addition, the preparation described in example 2-d has a highly reduced standard deviation referred to said parameters with respect to the reference product.

This result fully satisfies the objectives of the present patent application.

## Claims

1. Formulation of hydrophilic matrixes in the form of modified release tablets, based on substances endowed with antibiotic activity **characterized by**:
• a core containing, besides the active agent, hydrophilic polymeric materials controlling the release of the active substance and also adjuvants.

2. Formulation according to claim 1, provided with a coating made of polymeric materials soluble in water and/or aqueous liquids; said coating being applied only to hide the bitter taste of the active agent.

3. Formulations based on substances endowed with antibiotic activity according to claim 1, **characterized in that** they contain besides the antibiotic substance geleable and/or erodible hydrophilic polymers in presence of aqueous liquids, determining a modulation in the release of the transmitted active agent following a release kinetic pattern which can be pre-programmed "in vitro".

4. Formulations based on substances endowed with antibiotic activity according to claim 1, **characterized in that** after administration on healthy volunteers they can determine effective plasma levels enabling only one or two daily administrations, thus simplifying dosage and the correct use by the patient.

5. Formulations based on substances endowed with antibiotic activity according to claim 1, **characterized in that** the hydrophilic matrix carries a cephalosporin such as cephalotin, cephacetryl, cephapyrin, cephaloridin, cephalozin, cephaloglycine, cephalexin, cephadroxyl, cefaclor, cephadrin, cephatrizin, cephroxadin, cephuroxime axetil, cephamandol, cephonicid, cephuroxime, cephmetazol, moxalactam, cephotaxime, cephoperazone, cephoranid, cephpyramid, cephuzonam.

6. Formulations based on substances endowed with antibiotic activity according to claim 1, **characterized in that** said hydrophilic polymeric materials are chosen in the group comprising hydroxypropylmethyl cellulose with a molecular weight between 1,000 e 4,000,000, hydroxyethyl cellulose, methyl cellulose, polyvinyl pyrrolidone, carboxymethyl cellulose and its alkali metal or alkali-earth metal salts, carboxyvinyl polymers, polyvinyl alcohols, mannans, galactomannans, glucanes, scleroglucanes, carragenanes, pectine, xanthanes, agar, pullulane, poloxamers, polyoxyethylen glycols having different molecular weights, chitine, chitosanes and derivatives, beta cyclodextrin and derivatives of dextrins in general.

7. Formulations based on substances endowed with antibiotic activity according to claim 1, **characterized in that** hydroxypropylmethyl cellulose with the same average molecular weight can show different substitution degrees (and different relations between its substituents: ex. methoxyl-hydroxypropyl) such to give the polymer different properties of gelation and/or erosion and/or solution in contact with water and/or aqueous fluids.

8. Formulations based on substances endowed with antibiotic activity according to claim 1, **characterized in that** said hydrophilic polymeric materials are present in said core in a percentage between 5 and 90% of core weight, preferably between 10 and 50%.

9. Formulations based on substances endowed with antibiotic activity according to claim 1, **characterized in that** said hydrophilic polymeric materials according to claim 5 can be present as a single type or as a mixture of one or more polymers with different features of solubility, gelation and erosion.

10. Formulations based on substances endowed with antibiotic activity according to claim 1, **characterized in that** in the preparation of the hydrophilic matrix it is possible to employ a single type of hydrophilic polymer or mixtures of hydrophilic polymers having different features of gelation and/or erosion.

11. Pharmaceutical formulations according to claim 5, **characterized in that** the hydrophilic polymer is hydroxypropylmethyl cellulose having an average molecular weight of about 20,000 to 86,000.

12. Tablet according to claim 1, **characterized in that** said active substances are selected from the group comprising active agents having antibiotic activity such as cefaclor, cephamandol and cephalosporinic derivatives in general.

13. Tablet according to claim 2, **characterized in that** said polymeric coating is made of polymers such as hydroxypropylmethyl cellulose and other cellulose polymers with different solubility and/or permeability in contact with aqueous liquids.

14. Tablet according to claim 2, **characterized in that** said polymeric coating is applied in a turning tray following traditional methods and/or in a fluidized bed.

15. Tablet according to claim 2, **characterized in that** said polymeric coating constitutes 0.2 to 20% of the total weight of the finished system, preferably 3 to 15%.

16. Tablet according to claim 2, **characterized in that** said polymeric coating comprises plasticizers selected among castor oil, polyoxyethylen glycols with molecular weights of 400 to 20.000, hydrogenated castor oil, silicon derivatives such as simeticone, diethyl phtalate, triethyl citrate, tributyl citrate, triacetine, dibutyl sebacate.
